# EUROPEAN PATENT APPLICATION

(11) **EP 3 196 299 A1**
(43) Date of publication of application: **26.07.2017**
(21) Application number: 15841804.6
(22) Date of filing: 18.09.2015
(51) Int. Cl.: C12N 5/09, G01N 33/574

(54) **METHOD FOR THE DETECTION OF CIRCULATING TUMOUR CELLS, BOTH CIRCULATING TUMOUR CELLS OF THE EPITHELIAL PHENOTYPE AND CIRCULATING TUMOUR CELLS HAVING EPITHELIAL-MESENCHYMAL TRANSITION (EMT) MARKERS, USING THE MIRNA-21 AS A BIOMARKER**

(30) Priority: 18.09.2014 ES 201431357
(71) Applicant: Universidad de Granada, E-18071 Granada (ES); Servicio Andaluz de Salud, E-41071 Sevilla (ES)
(72) Inventor: SERRANO FERNÁNDEZ, María José, 18071 Granada (ES); DÍAZ MOCHÓN, Juan José, 18071 Granada (ES); GABRIEL ORTEGA, Francisco, 18012 Granada (ES); LORENTE ACOSTA, José Antonio, 18071 Granada (ES); GARCÍA PUCHE, José Luis, 18012 Granada (ES); RUIZ BLAS, María Paz, 18071 Granada (ES); SÁNCHEZ MARTÍN, Rosario María, 18071 Granada (ES)
(74) Representative: Hoffmann Eitle
(86) International application number: PCT/ES2015/070681
(87) International publication number: WO 2016/042197

(57) **Abstract**

The present invention provides an in vitro method of detecting circulating tumour cells, circulating tumour cells of epithelial phenotype and circulating tumour cells of epithelial to mesenchymal transition (EMTs), in a biological sample using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumour cells in said biological sample or wherein if the expression levels in the cells of the biological sample are expressed in an amount greater than 2/3 of the maximum expression achieved in a positive control is indicative of the presence of circulating tumour cells in said biological sample.

SEQUENCE LISTING

<110>  UNIVERSIDAD DE GRANADA

<120>  Procedure to detect circulating tumor cells, both circulating
       tumour cells of epithelial phenotype and circulating tumour cells
        having Epithelial-mesenchymal transition markers (EMTs), by
       using miRNA-21 as a biomarker.

<130>  902166

<160>  1

<170>  PatentIn version 3.5

<210>  1
<211>  22
<212>  RNA
<213>  Artificial Sequence

<220>
<223>  hsa-miRNA-21



<220>
<221>  misc_feature
<222>  (1)..(22)
<223>  hsa-miRNA-21

<400>  1
uagcuuauca gacugauguu ga                                              22

## Description

### TECHNICAL FIELD OF THE INVENTION

The present invention refers to the medical field, in particular to a procedure to detect circulating tumor cells, both circulating tumor cells of epithelial phenotype and circulating tumor cells having Epithelial-mesenchymal transition markers (EMTs), by using miRNA-21 as a biomarker.

### STATE OF THE ART/BACKGROUND OF THE INVENTION

### Circulating tumor cells

Metastasis is responsible for the vast majority of cancer-related deaths. During this process, circulating tumor cells (CTC) are generated, spread from the primary tumor, colonize distant organs and lead to overt metastatic disease. During the past decade a growing interest in CTCs has spread out across the oncology field, especially looking at their capacity as prognostic elements of cancer.

### CTCs detection

Despite important progresses in understanding and detecting CTCs, most of the assays still have low sensitivity; mainly due to the use of a few epithelial biomarkers to identify and isolate them from whole blood. EpCam and/or cytokeratins (CK) are the two main epithelial biomarkers included in most of the devices used to date. Amongst those devices CellSearch and GILUPI, which have been approved by the FDA and the EU as medical devices respectively, are based on detecting just EpCam on circulating cells in blood.

However, recent evidences have demonstrated that a subset of CTCs may lack EpCAM and cytokeratin expression and instead exhibit epithelial-mesenchymal transition (EMT) features. Additionally, by using epithelial biomarkers, it is thus possible to identify epithelial cells within hematopoietic cell populations which are not coming from tumor but from other epithelial tissues. Accordingly, the development of novel detection platforms should be accompanied by novel and specific CTC biomarkers that enhance their detection and molecular characterization.

### MicroRNAs

MicroRNAs (miRNAs) are small non-coding RNAs which play a key role in the post-transcriptional regulation of mRNA. Variations in miRNA expressions related to different pathologies, including different kinds of cancer, have been described in many reports. miRNAs also circulate within body fluids, including peripheral blood and urine, with many studies reporting correlation between levels of specific circulating miRNAs and different pathologies, specially cancer. Therefore, they have been proposed as ideal biomarkers to develop diagnostic and prognostic liquid biopsy assays. However, technical difficulties to do robust and comparable profiling of circulating miRNAs across different platforms as well as inter-personal variability, lack of common internal normalizers and their unclear functional roles have impacted negatively so far in succeeding to develop an approved clinical diagnostic assay based on them.

To date, different efforts to correlate circulating miRNAs and number of CTCs have also been done. Moreover, in 2011, Sieuwerts profiled miRNAs from lysates of blood fractions containing CTCs. However, that approach might be challenging to be broadly implemented due to the low numbers of CTCs present in blood and the inherited issue of leukocyte contamination.

### BRIEF DESCRIPTION OF THE INVENTION

The present invention confronts the problem of providing an efficient and sensitive method to detect CTCs, both circulating tumor cells of epithelial phenotype and circulating tumor cells having Epithelial-mesenchymal transition markers (EMTs), in a biological sample. In this sense, we herein provide a solution to this problem by using a methodology based on the expression level of miRNA-21 as a biomarker for the detection of circulating tumor cells, both types above mentioned, preferably combined with immunomagnetic selection and/or immunocytochemistry.

Particularly, the present invention provides an *in vitro* method of detecting circulating tumour cells, circulating tumour cells of epithelial phenotype and circulating tumour cells of epithelial to mesenchymal transition (EMTs), in a biological sample using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumour cells in said biological sample or wherein if the expression levels in the cells of the biological sample are expressed in an amount greater than 2/3 of the maximum expression achieved in a positive control is indicative of the presence of circulating tumour cells in said biological sample.

### BRIEF DESCRIPTION OF THE FIGURES

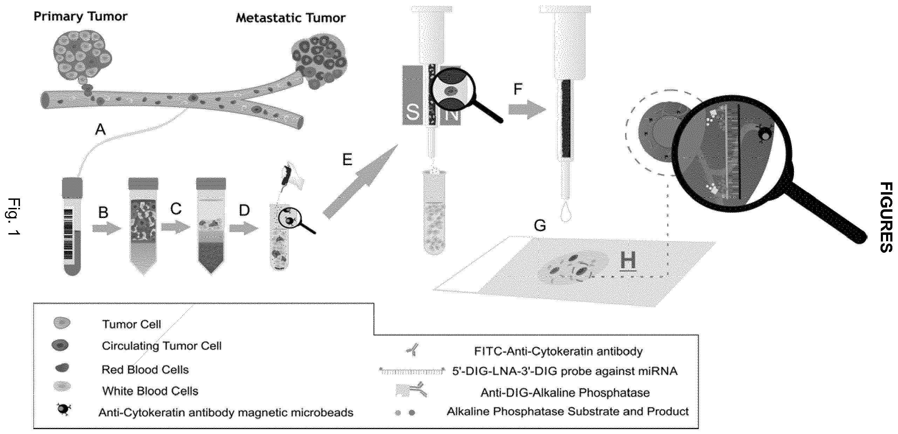

**Figure 1****.** Schematic illustration of the MishCTCsmethod for simultaneous miRNA and CK immunocytochemistry detection. (A) recovery of peripheral blood into an EDTA tube; (B) blood transfer into a density-gradient centrifuge tube; (C) centrifugation at 700g for 30 min; (D) recovery of interphase containing mononuclear and tumor cells and immunomagenetic labeling with anti-CK antibody magnetic microbeads; (E) magnetic cell separation assisted by a MiniMACS separator using a pre-filled separation column; (F) elution of retained cells; (G) cytospin onto poly-lysine glass slide and (H) MishCTC detection of miRNA and CK.

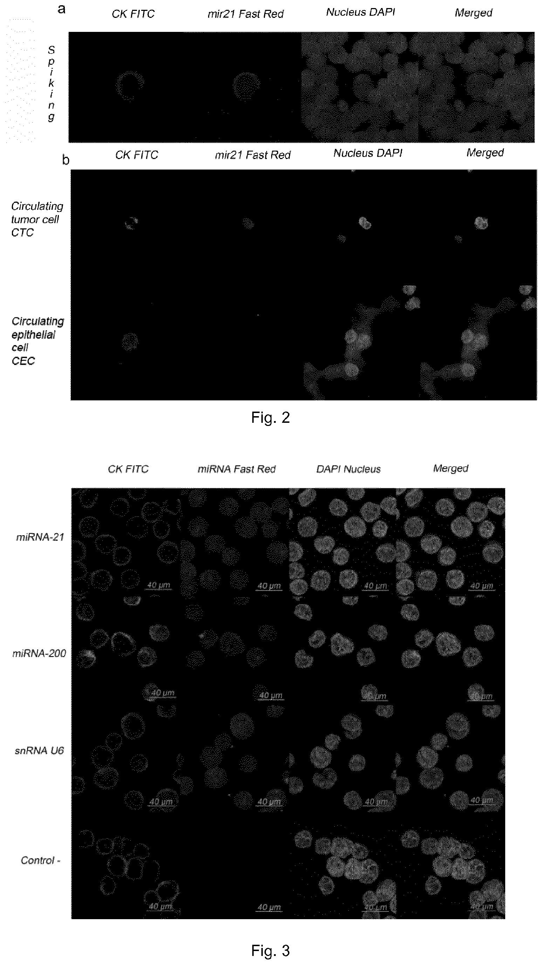

**Figure 2****.** Image galleries obtained with MishCTC method (A) CK and miRNA-21 expression in a CTC isolated from a metastatic lung cancer patient following MishCTC methods. All CTCs found within this set of patients were both CK and miRNA positives. (B) CK expression in a circulating epithelial cell found in a cancer-free patient undergoing a nephrectomy operation. CK protein expression (green) was detected by immunofluorescence but miRNA21 could not be detected by in situ hybridization.
**Figure 3****.** Summary of fluorescent images showing miRNA sequences and cytokeratins expressed in MDA-MB468 tumor cell line using locked nucleic acid (LNA) probes labeled with digoxigenin and anti-CK antibody FITC. miRNA and cytokeratin were thus detected by both in situ hybridization of miRNA and immunofluorescence technique. Rows show cytokeratin, miRNAs, nuclei (Dapi) and merged images. Each row corresponds to detection of miRNA-21, miRNA-200, snRNA U6 and control (none LNA
   3 probe was added) from top to bottom. LNA™ scrambled microRNA probe, double-DIG labeled. LNA™ (5'-gtgtaacacgtctatacgccca-3') was used as negative control.

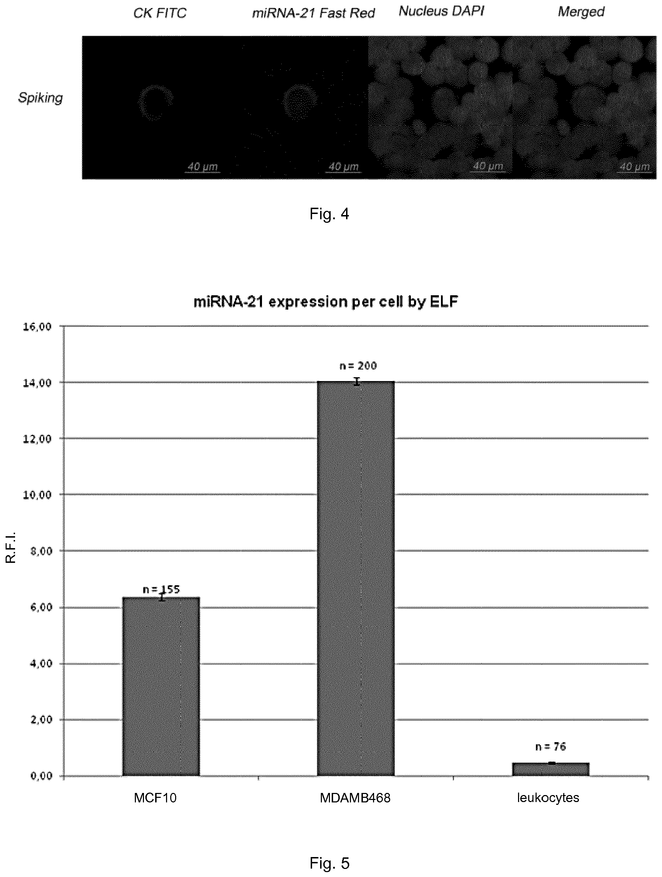

**Figure 4****.** Image galleries obtained with MishCTC method. CK and miRNA-21 expression in a MDA-MB468 cell which was spiked into a healthy volunteer blood sample. Detection of cytokeratin-positive (CK+) cell (green channel), miRNA-21 (red channel) and nuclei (blue channel). Epithelial cell was identified amongst a leukocyte population which did not expressed miRNA-21.
**Figure 5****.** Mean fluorescence intensities of miRNA21 in MDA-MB468, MCF10A, and leucocytes generated by ELF signal amplification using LNA probes. Quantification was performed using Image J software. miRNA-21 was over-expressed in MDAMB468 cell tumor line if compared with epithelial non tumor cell line MCF10A. None fluorescence signal was observed in leucocytes.

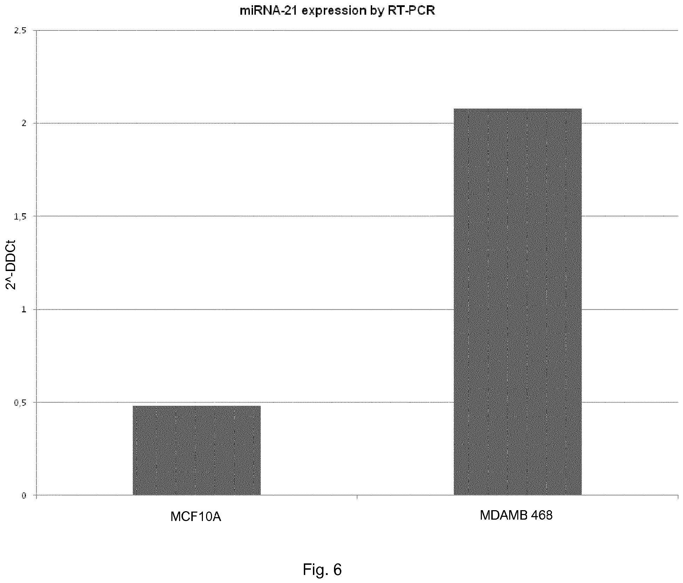

**Figure 6****.** Expression of miRNA-21 by RT-PCR. These experiments showed a relative higher expression of miRNA21 in MDA-MB468 than in MCF10A. The molecular analysis by RT-PCR corroborates the potential value of miRNA21 to differentiate circulating epithelial tumor cells from epithelial non-tumor cells.

### DETAILED DESCRIPTION OF THE INVENTION

### Definitions

In the context of the present invention, the following terms have the following meanings:
As used herein, miRNA-21 is understood as hsa-miRNA-21 having the following nucleotide sequence 5'-uagcuuaucagacugauguuga-3'. Synthetic probes preferably used to detect hsa-miRNA-21, contain all or part of the following sequence 5'-TCAACATCAGTCTGATAAGCTA-3'. Synthetic probes might be based on LNA, DNA, RNA, BNA or PNA.

As used herein, for comparing the expression levels of miRNA-21 between cells of a biological sample with a negative control or with a positive control the following steps are preferably taken: measuring expression of miRNA by rtPCR using SYBR Green and a normalizer, for example, U6 RNA or miRNA16. Measuring miRNA expression by fluorescence microscopy upon Enzyme-Labeled Fluorescence (ELF) Signal. Amplification step.

As used herein, the labelled-LNA probes used to detect miRNA-21 is sequence 5'-TCAACATCAGTCTGATAAGCTA-3' labeled at both ends with digoxin. Following in-situ hybridization protocols, digoxin is then recognized by anti-digoxin antibody labeled with alkaline-phosphatase which upon reaction with FastRed substrate produce a fluorescence insoluble product.

As used herein MishCTC is understood as detection of miRNA by in-situ hybridization in circulating tumoral cells (CTC)

As used herein in situ hybridization (ISH) is understood as a technique for identifying single strand miRNA within individual cells.

As used herein circulating tumor cells is understood as cells found within the bloodstream coming from a primary epithelial tumor and circulate.

As used herein circulating tumor cells of epithelial phenotype is understood as cells found within the bloodstream coming from a primary epithelial tumor which keep their epithelial markers such as EpCam and cytokeratine.

As used herein circulating tumour cells having Epithelial-mesenchymal transition markers (EMTs) is understood as cells found within the bloodstream coming from a primary epithelial tumor which have changed their epithelial phenotype losing some of the epithelial markers such EpCam and cytokeratine while expressing mesenchymal markers such as SNAIL and vimentine.

As used herein malignant progression is understood as indication of either lack of response to chemotherapeutic and/or biological treatments or even aggravation of health condition of the cancer patient.

### Description of the invention

The present invention provides new *in vitro* methods of detecting circulating tumor cells, both circulating tumour cells of epithelial phenotype and circulating tumor cells having Epithelial-mesenchymal transition markers (EMTs), in a biological sample using, as an indicator, expression levels of miRNA-21.

In this sense, the authors of the present invention have discovered a novel procedure or protocol to detect circulating tumor cells (CTCs) in a patient's blood sample by *in situ* hybridization of specific miRNAs (MishCTC) which is compatible with simultaneous immunocytochemistry protocols for cell phenotyping. To our knowledge this is the very first time that a procedure to identify miRNAs in CTCs by in-situ hybridization techniques has been reported.

To detect miRNAs in CTCs the authors integrated ISH *(in situ* hybridization) protocols for detecting miRNAs in single cells with methodological steps required to isolate and identify CTCs coming from a patient's blood sample. Initial experiments were carried out using an epithelial tumor breast cell line as a model cell line. Briefly, following cell collected from well-plates, cells were placed on slides by cytospin. Cells were then treated with 1-Ethyl-3-(3-dimethylaminopropyl)carbodiimide (EDC) in order to covalently immobilized microRNAs to cytoplasm. Detection was done by an enzyme-labeled fluorescence (ELF) signal amplification approach using miRCURY technology which is based on LNA probes. This technology uses labeled-LNA probes which hybridized their fully complementary miRNA sequences with high-affinity. Those tags can be consequently revealed via antibodies labeled with enzymes which convert fluorogenic enzymatic substrates into fluorescent products. Herein, digoxin (DIG) and sheep anti-DIG antibody labeled with alkaline phosphatase were used as partners and FastRed TR as fluorogenic substrate. Upon alkaline phosphate enzymatic activity, FastRed TR substrate produces an insoluble product that can be detected by fluorescence microscopy (Fig. 1).

Epithelial tumor breast cell line (MDA-MB468) (ATCC® HTB-132™) was used as a model to detect *in-situ* miRNA 200, miRNA21 and U6 by fluorescence microscopy. Apart from RNAs, nuclei and cytokeratins were also stained by DAPI and anti-cytokeratin antibodies labeled with FITC, respectively (Fig. 3 shows fluorescence images obtained by this methodology). Cell rounded morphologies and miRNA distributions are concordant with cytospin treatments and with LNA-based ELF detection respectively. This protocol, which successfully detected RNA via ELF signal amplification in cells placed on slides by cytospin was then applied for the rest of experiments.

The very low number of CTCs in blood, compared with the number of the hematopoietic cells, is one of the most challenging aspects for any technology focused on molecular characterization. In order to optimize the methodological steps required to isolate CTCs from blood via positive selection of CTCs with epithelial phenotype and miRNA detection, the authors of the present invention spiked fifteen healthy volunteer blood samples with 100 epithelial cells MDA-MB468 each. Isolation of cytokeratin-positive cells and their further phenotypic characterization were based on the protocol established in the examples (see Methods, Fig.1). After placing cytokeratin-positive cell fractions onto slides by cytospin, the authors followed the same protocol described above to identify miRNA-21. The authors chose miRNA-21 for our *in situ* experiments as it has been described as one of the most important miRNAs related to cancer development. Moreover, the chosen miRNA has an important feature, namely the fact that it is expressed in tumor cells but not in hematopoietic cells so that CTCs and leukocytes can be easily differentiated. In addition, miRNA-21 might also be able to differentiate CTCs from epithelial normal cells as their expression level might be different. On average, the authors isolated a 79% of the total number of spiked cells and, in all samples, every cell, which was cytokeratin positive, also expressed miRNA-21 without exception (see Table 1).

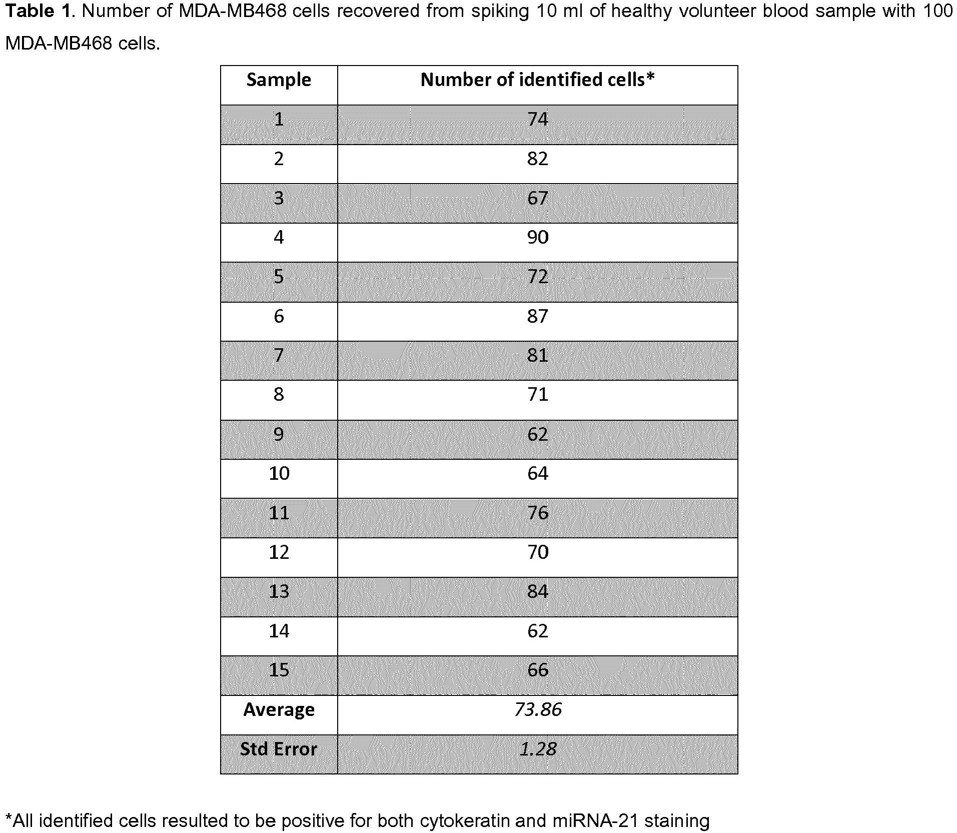

Fig. 4 shows images from a spiking experiment where a single MDA-MB468 is detected amongst the leukocyte population. MiRNA-21 is clearly identified in epithelial cells while it is not detected in leukocytes and therefore fulfills one of the most important requirements for this assay. These data demonstrate that miRNA-21 expression can be considered as a specific biomarker for epithelial cells which does not appear in hematopoietic lineage.

Before proceeding with the analysis of cancer patient blood samples the authors decided to investigate whether miRNA-21 was expressed equally in tumor and non-tumor epithelial cells. They thus performed both *in-situ* hybridization of miRNA-21 according to the previously established protocols and miRNA-21 expression analysis by qRT-PCR in breast cancer cell line (MDAMB468) and normal breast cell line (MCF10A). Fluorescence intensities per cell and ddCT values from both cell lines confirmed that miRNA-21 is over-expressed in epithelial tumour cells if compared to non-tumour cells (see examples and Fig. 5 and Fig. 6). These results thus confirm miRNA-21 as a perfect biomarker to differentiate CTCs from leukocytes and tumor from non-tumor epithelial cells.

To evaluate the potential implementation of this method as diagnostic tool in patient blood samples, the authors used peripheral blood samples from 25 oncologic metastatic patients, with informed consent (see Table 2).

**Table 2. Anatomic pathological characteristics of cancer patients and number of circulating epithelial cells with expressing CK and miRNA-21.**

| **Patient Code** | **TUMOR TYPE** | **METASTATIC TISSUE** | **HISTOLOGY** | **CK+ miRNA-21 -** | **CK+miRN A-21+** |
|---|---|---|---|---|---|
| **1** | COLON | LIVER | ADENOCARCINOMA | 0 | 3 |
| **2** | BREAST | BONE | ADENOCARCINOMA | 0 | 0 |
| **3** | MELANOME | SKIN | | 0 | 0 |
| **4** | LUNG | BONE | SQUAMOUS CELL CARCINOMA | 0 | 0 |
| **5** | COLON | TORAX AND LIVER | ADENOCARCINOMA | 0 | 0 |
| **6** | LARINGE | LYMPH NODES | SQUAMOUS CELL CARCINOMA | 0 | 1 |
| **7** | BREAST | THORACIC WALL | CDI GII | 0 | 0 |
| **8** | COLON | LUNG | ADENOCARCINOMA GII | 0 | 0 |
| **9** | OVARY | ABDOMEN | SEROUS CARCINOMA | 0 | 0 |
| **10** | COLON | ILIAC CREST | MUCOSECRETOR ADENOCARCINOMA | 0 | 0 |
| **11** | COLON | OVARY AND UTERUS | ADENOCARCINOMA GII | 0 | 2 |
| **12** | COLON | LIVER | ADENOCARCINOMA | 0 | 1 |
| **13** | LUNG | PLEURAL CAVITY | ADENOCARCINOMA | 0 | 0 |
| **14** | OVARY | ABDOMEN | INDIFFERENT CARCINOMA | 0 | 1 |
| **15** | PROSTATE | BONE AND MEDIASTINUM | ADENOCARCINOMA | 0 | 7 |
| **16** | GASTRIC | LIVER | ADENOCARCINOMA GII | 0 | 0 |
| **17** | BREAST | BONE | CLI | 0 | 0 |
| **18** | COLON | LIVER AND LUNG | ADENOCARCINOMA GIII | 0 | 0 |
| **19** | LUNG | MEDIASTINUM | SQUAMOUS CELL | 0 | 6 |
| **20** | OVARY | LUNG AND LYMPH NODES | MIXED EPITHELIAL ADENOCARCINOMA | 0 | 2 |
| **21** | COLON | LIVER | ADENOCARCINOMA | 0 | 2 |
| **22** | LUNG | LYMPH NODES | SMALL CELL LUNG CARCINOMA | 0 | 0 |
| **23** | COLON | LUNG AND LIVER | MUCOSECRETOR ADENOCARCINOMA | 0 | 1 |
| **24** | UROTHELIAL | ABDOMEN AND LYMPH NODES | PAPILLARY UROTHELIAL CARCINOMA | 0 | 1 |
| **25** | ADRENAL | LIVER AND LUNG | ADRENOCORTICAL CARCINOMA | 0 | 0 |

All the samples, including samples from healthy donors, were treated and analyzed as described above. Within this patient population, CTCs were detected in 10 patients and in all cases CTCs were identified concomitantly by CK and miRNA-21 expression (Fig. 2a). All samples from healthy donors were negative for both CK and miRNA21 expression. With this method we can thus determinate the number of CTCs in oncologic patients, using miRNA-21 as biomarker. Finally, and in order to confirm that miRNA-21 is expressed differently in circulating tumor and non-tumor epithelial cells, a blood sample taken from a cancer-free patient who just underwent a nephrectomy operation was used as source of circulating non tumor epithelial cells. Fig. 2b shows microscopy images obtained from that sample following our MishCTC protocol. In this case epithelial cell did not show miRNA-21 expression while keeping its epithelial cytokeratin phenotype.

In summary, we herein introduce the first methodology for *in-situ* hybridization of miRNA in CTCs with epithelial phenotype which is compatible with immunocytochemistry detection. In this sense, we herein report that expression of miRNA-21 is restricted to the epithelial tumor cells detected in peripheral blood whereas miRNA-21 is either under-expressed in circulating non-tumor epithelial cells and non-tumor epithelial cell lines or absent in leukocytes. MishCTC results also revealed that miRNA-21 is consistently over-expressed in CTCs coming from metastatic solid tumours. This discovery is the first proof of concept for MishCTC and it can be applied to analyze multi-miRNAs in CTCs as potential tool to monitor cancer patients and their treatment efficiency. These protocols are also useful to gain a better understanding of the molecular mechanisms associated with dissemination and metastatic processes.

Thus a first aspect of the invention refers to an in vitro method of detecting circulating tumour cells, both circulating tumour cells of epithelial phenotype and circulating tumour cells having Epithelial-mesenchymal transition markers (EMTs), in a biological sample using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumor cells in said biological sample or wherein if the expression levels in the cells of the biological sample are expressed in an amount greater than 2/3 of the maximum expression achieved in a positive control is indicative of the presence of circulating tumour cells in said biological sample.

In the context of the present invention illustrative non-limiting examples of a biological sample include different types of samples from tissues, as well as from biological fluids, such as blood, serum, plasma, cerebrospinal fluid, peritoneal fluid, faeces and urine. Preferably, said samples are samples from tissues and most preferably, said samples of tissues originate from tumour tissue of the individual the response of which is to be predicted, and may originate from biopsies.

In another preferred embodiment, the expression levels of miRNA-21 are determined by *in-situ* hybridization.

In another preferred embodiment, the negative control is a non-tumor epithelial cell or a hematopoietic cell and wherein as used herein overexpression is meant an at least two fold expression level of miRNA-21 in the cells of the biological sample in comparison to the expression level of miRNA-21 in a non-tumour epithelial cell as determined by *in-situ* hybridization or an at least 10 fold expression level of miRNA-21 in the cells of the biological sample in comparison to the expression level of miRNA-21 in a hematopoietic cell, preferably lymphocytes or mononuclear cells or leukocytes, as determined by *in situ* hybridization.

In another preferred embodiment, the positive control is the epithelial tumor breast cell line (MDA-MB468).

In another preferred embodiment, the biological sample is first treated to isolate the cytokeratin positive cells and/or the EpCAM positive cells and/or SNAIL positive cells and/or vimentine positive cells, wherein preferably the cytokeratin positive cells and/or the EpCAM positive cells and/or the SNAIL positive cells and/or the vimentine positive cells are isolated by immunomagnetic selection and/or immunocytochemistry.

The methods of the present invention may be applied with samples from human individuals of either sex, i.e. men or women, and at any age. The profile determined by the present invention might be diagnostic, predictive and prognostic.

Thus a second aspect of the invention refers to a method of predicting or prognosticating the progression of cancer in a biological sample of a subject, wherein the subject is suffering from a cancer disease, and wherein the method comprises using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of a malignant progression of said cancer disease or wherein if the expression levels in the cells of the biological sample are expressed in an amount greater than 2/3 of the maximum expression achieved in a positive control is indicative of a malignant progression of said cancer disease.

In addition, a third aspect of the invention refers to a method of diagnosing cancer in a subject, wherein the method comprises using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumour cells in said biological sample or wherein if the expression levels in the cells of the biological sample are expressed in an amount greater than 2/3 of the maximum expression achieved in a positive control is indicative of the presence of circulating tumour cells in said biological sample.

In a preferred embodiment of any of the second or third aspect of the invention, the subject is a human subject.

In another preferred embodiment of any of the second or third aspect of the invention, the biological sample is any biological fluid. In the context of the present invention illustrative non-limiting examples of a biological sample include different types of samples from tissues, as well as from biological fluids, such as blood, serum, plasma, cerebrospinal fluid, peritoneal fluid, faeces and urine. Preferably, said samples are samples from tissues and most preferably, said samples of tissues originate from tumour tissue of the individual the response of which is to be predicted, and may originate from biopsies.

In another preferred embodiment of any of the second or third aspect of the invention, the expression levels of miRNA-21 are determined by in-situ hybridization.

In another preferred embodiment of any of the second or third aspect of the invention, the negative control is a non-tumor epithelial cell or a hematopoietic cell and wherein as used herein overexpression is meant an at least two fold expression level of miRNA-21 in the cells of the biological sample in comparison to the expression level of miRNA-21 in a non-tumor epithelial cell as determined by *in situ* hybridization or an at least 10 fold expression level of miRNA-21 in the cells of the biological sample in comparison to the expression level of miRNA-21 in a hematopoietic cell, preferably lymphocytes or mononuclear cells, as determined by in situ hybridization.

In another preferred embodiment of any of the second or third aspect of the invention, the positive control is the epithelial tumor breast cell line (MDA-MB468).

In another preferred embodiment of any of the second or third aspect of the invention, the biological sample is first treated to isolate the cytokeratin positive cells and/or the EpCAM positive cells and/or SNAIL positive cells and/or vimentine positive cells. Preferably the cytokeratin positive cells and/or the EpCAM positive cells and/or the SNAIL positive cells and/or the vimentine positive cells are isolated by immunomagnetic selection and/or immunocytochemistry.

In another preferred embodiment of any of the second or third aspect of the invention, the cancer disease is a solid tumor of epithelial origin. Preferably the solid tumor of epithelial origin is selected from the list consisting of ovarian, head and neck, larynx, colon, stomach, prostate, cervix, gastric, urothelial, adrenal, thyroid gland, lung, uterus, rectum, breast or kidney cancer or carcinoma or a sarcoma, melanoma.

In the context of the present invention the method of determining the expression level of miRNA-21 need not be particularly limited, and may be selected from method comprising PCR, such as real time PCR; and/or an in-situ hybridization assay.

Real time quantitative PCR (RQ-PCR) is a sensitive and reproducible gene expression quantification technique which can particularly be used to profile miRNA expression in cells and tissues. Without prejudice of the method used to determine the response (RQ-PCR, *in situ* hybridization etc..), in the context of the present invention a "significantly increased expression" or "over-expression" can be defined in comparison to a negative sample and/or to a positive control.

In this sense, a "negative sample" or a "sample of reference" is defined as a sample that does not express or has a basal level of expression of miRNA-21, i.e. a non tumoral epithelial cell sample originating from the same tissue of the biopsy of origin (in the case of lung cancer the control sample would be non-tumoral lung tissue). Another example would be any type of non-tumoral hematopoietic cell such as leukocyte, lymphocytes or mononuclear cells.

In the context of the present invention a positive control sample is epithelial tumor breast cell line MDA-MB468.

Additionally, the present inventors have identified a novel subgroup of patients that will profit from chemotherapy and/or radiotherapy. Hence, a further aspect of the invention refers to a method for allocating a human subject suffering from cancer in one of two groups, wherein group 1 comprises subjects identifiable by the method according to any of the previous aspects; and wherein group 2 represents the remaining subjects.

A still further aspect of the invention refers to a pharmaceutical composition comprising a chemotherapeutic drug such as cisplatin and/or hycamtin for treating a human subject of group 1 as identifiable by the method of the previous aspect of the invention.

In a particular embodiment of the invention, the treatment of choice of a human subject suffering from cancer of group 1, as identifiable by the method of the previous aspect of the invention, includes but is not limited to the following types: radiotherapy, platinum coordination complexes, doxorubicin and other antracycins, bortezomib, campothecin, procarbazine, cyclophosphamide, adriamycin or alkylating agents, photodynamic therapy and biologicals such as rituximab.

Yet a further aspect of the invention refers to a pharmaceutical composition comprising platinum coordination complexes, doxorubicin and other antracycins, bortezomib, campothecin, pro-carbazine, cyclophosphamide, adriamycin or alkylating agents, photodynamic therapy and biologi-cals such as rituximab, for treating a human subject of group 1 as identifiable by the method of the former aspect of the invention.

The present invention also provides a kit or a device suitable to put into practice the method of the invention, comprising at least one oligonucleotide(s) capable of hybridizing with miRNA-21 and optionally means for the detection of cytokeratin positive cells by immunomagnetic selection and/or immunocytochemistry. Preferably, the kit further comprises a positive control sample, optionally a non-tumour epithelial cell.

The kit is based on the prognostic, predictive and diagnostic power of the method of the present invention. It is preferred that said oligonucleotide(s) hybridizes with two mismatches or less, and preferably with no mismatch, with respect to the miRNA to be determined. As far as hybridization of the oligonucleotide(s) is concerned, it is preferred that said oligonucleotide(s) is capable to do so under stringent conditions. Stringency is a term used in hybridization experiments. Stringency reflects the degree of complementarity between the oligonucleotide and the nucleic acid (which is in this case the mRNA to be detected); the higher the stringency, the higher percent homology between the probe and filter bound nucleic acid. It is well known to the skilled person that the temperature and salt concentrations have a direct effect upon the results that are obtained. It is recognized that the hybridization results are related to the number of degrees below the Tm (melting temperature) of DNA at which the experiment is performed. Often, stringent conditions are defined as a wash with 0.1X SSC (saline-sodium citrate (SSC) buffer at 65°C. (SSC is typically provided as 20X stock solution, which consists of 3 M sodium chloride and 300 mM trisodium citrate (adjusted to pH 7.0 with HCl)).

The kit or device may be used and the use is not particularly limited, although use in the method of the invention in any of its embodiments is preferred.

Another aspect of the invention relates to a computer readable storage medium/data carrier comprising the program according to the third aspect of the invention, the computer program performing the steps of any one of the methods of the invention.
The medium in which the computer program is encoded may also comprise transmission signals propagating through space or a transmission media, such as an optical fiber, copper wire, etc. The transmission signal in which the computer program is encoded may further comprise a wireless signal, satellite transmission, radio waves, infrared signals, Bluetooth, etc. The transmission signal in which the computer program is encoded is capable of being transmitted by a transmitting station and received by a receiving station, where the computer program encoded in the transmission signal may be decoded and stored in hardware or a computer readable medium at the receiving and transmitting stations or devices.

Another aspect of the invention relates to a transmission signal comprising program instructions capable of causing a computer to perform the steps of any one of the methods of the invention.

A last aspect of the present invention relates to the use of miRNA-21 for detecting circulating tumour cells, both circulating tumour cells of epithelial phenotype and circulating tumour cells having Epithelial-mesenchymal transition markers (EMTs), preferably in a biological sample.

The following examples serve to illustrate the present invention; these examples are in no way intended to limit the scope of the invention.

### EXAMPLES

### Example 1. Integration of LNA-based miRNA-ISH techniques and CTC protocols.

To detect miRNAs in CTCs the authors integrated ISH protocols for detecting miRNAs in single cells with methodological steps required to isolate and identify CTCs coming from patient blood. Initial experiments were carried out using an epithelial tumor breast cell line as model. Following cell collected from well-plates, cells were placed on slides by cytospin. Cells were then treated with EDC in order to covalently immobilized miRNAs to cytoplasm. Detection was done by an enzyme-labeled fluorescence (ELF) signal amplification approach using miRCURY technology which is based on LNA probes. This technology uses labeled-LNA probes which hybridized their fully complementary miRNA sequences with high-affinity. Those tags can be consequently revealed via antibodies labeled with enzymes which convert fluorogenic enzymatic substrates into fluorescent products. Herein, digoxin (DIG) and sheep anti-DIG antibody labeled with alkaline phosphatase were used as partners and FastRed TR as fluorogenic substrate. Upon alkaline phosphate enzymatic activity, FastRed TR substrate produces an insoluble product that can be detected by fluorescence microscopy (Fig. 1).

Epithelial tumor breast cell line (MDA-MB468) was used as model to in-situ detect miRNA 200, miRNA21 and U6 by fluorescence microscopy. Apart from RNAs, nuclei and cytokeratins were also stained by DAPI and anti-cytokeratin antibodies labeled with FITC, respectively (SI Fig 1 shows fluorescence images obtained by this methodology). Cell rounded morphologies and miRNA distributions are concordant with cytospin treatments and with LNA-based ELF detection respectively. This protocol, which successfully detected RNA via ELF signal amplification in cells placed on slides by cytospin was then applied for the rest of experiments.

### Example 2. Detection of miRNA-21 in MDA-MB468 cell lines spiked in blood samples from healthy volunteer.

The very low number of CTCs in blood, compared with the number of the hematopoietic cells, is one of the most challenging aspects for any technology focused on molecular characterization. In order to optimize the methodological steps required to isolate CTCs from blood, via positive selection of CTCs with epithelial phenotype, and then detect miRNAs, the authors spiked fifteen healthy volunteer blood samples with 100 epithelial cells MDA-MB468 each. Isolation of cytokeratin-positive cells and their further phenotypic characterization was based on the protocol establish in the Materials and Methods of the present invention and in Fig. 1. After placing cytokeratin-positive cell fractions onto slides by cytospin, the authors followed the same protocol described above to identify miRNA-21. On average, the authors isolated a 79% of the total number of spiked cells and, in all samples, every cell, which was cytokeratin positive, also expressed miRNA-21 without exception (see Table 1). Fig. 2a shows images from a spiking experiment where a single MDA-MB468 is detected amongst the leukocyte population. MiRNA-21 is clearly identified in epithelial cells while it is not detected in leukocytes and therefore fulfill one of the most important requirements for this assay. These data demonstrate that miRNA-21 expression can be considered as a specific biomarker for epithelial cells which does not appear in hematopoietic lineage.

### Example 3. Quantification of miRNA-21 in MDA-MB468 and MCF-10 cell lines by qRT-PCR.

Before proceeding with the analysis of cancer patient blood samples the authors decided to investigate if miRNA-21 was expressed equally in tumor and non-tumor epithelial cells. The authors thus performed both in-situ hybridization of miRNA-21 according to the protocols established before and miRNA-21 expression analysis by qRT-PCR in breast cancer cell line (MDA-MB468) and normal breast cell line (MCF10A). Fluorescence intensities per cell and ddCT values from both cell lines confirmed that miRNA-21 is over-expressed in epithelial tumor cells if compared to non-tumor cells (see Methods and Fig. 4 and Fig. 5). These results thus confirm miRNA-21 as a valid biomarker to differentiate CTCs from leukocytes and tumor from non-tumor epithelial cells.

### Example 4. Detection of miRNA-21 in CTCs from blood samples of cancer patients.

To evaluate the potential implementation of this method as diagnostic tool in patient blood samples, the authors used peripheral blood samples from 25 oncologic metastatic patients, with informed consent (see Table 2). All the samples, including samples from healthy donors, were treated and analyzed as described above. Within this patient population, CTCs were detected in 11 patients and in all cases CTCs were identified concomitantly by CK and miRNA-21 expression (see Fig. 2b and Table 2). All samples from healthy donors were negative for both CK and miRNA21 expression. Finally, and in order to confirm that miRNA-21 is expressed differently in circulating tumor and non-tumor epithelial cells, a blood sample taken from a cancer-free patient who just underwent a nephrectomy operation was used as source of circulating non tumor epithelial cells. Fig. 2b shows microscopy images obtained from that sample following our MishCTC protocol. In this case epithelial cell did not show miRNA-21 expression while keeping its epithelial cytokeratin phenotype.

### Example 5. Detection of miRNA-21 in EMT-induced MCF-7 cell lines.

The authors used the MishCTC protocol for investigating miRNA-21 expression in EMT-induced MCF-7 cell lines, a tumor epithelial cell line, as a model of cell heterogeneity which is found within CTCs. The authors thus tried to see if the method described herein could detect heterogeneous epithelial cell lines which were losing epithelial biomarkers such as cytokeratin and kept expressing miRNA-21. MCF7 cells were plated in 96 well-plates and induced by TGF-□□. Fig. 6 shows miRNA-21 and CK expression in both MCF-7 and TGF-□ □ induced MCF-7 cell lines labeled using the MishCTC protocol. It is worth noting that unmodified MCF-7 cell lines expressed miRNA-21 in an heterogeneous manner within the same cell culture, a heterogeneity which was not seen previously in MDA-MBA468. In the case of TGF-□□□induced MCF-7 cell lines, and as expected, there was a population of cells which lost CK expression but maintained miRNA-21, giving rise to cells which were CK negative and miRNA-21 positives.

### Example 6. Materials and Methods.

All experiments were performed in accordance with relevant guidelines and regulations.

### Cell culture.

Breast cancer cell lines were obtained from American Type Cell Culture Collection of Cell Cultures (ATCC, Manassas, USA). MDA-MB468 tumor cells were maintained in DMEN culture medium (Gibco, UK) supplemented with 10 % fetal bovine serum (Gibco, UK) and 100 U ml-1 of penicillin and 100 ng ml-1 of streptomycin at 37°C in 5% humidified CO2 incubator. MCF10-A non-tumor cells were maintained in mammalian epithelial growth medium (MEGM) serum free (Clonetics® Lonza, New Jersey, USA) with 100 ng mL-1 of choleric toxin (Sigma Aldrich, USA)

### Preparation of Cytospins from breast cancer cell lines for simultaneous detection of cytokeratines and miRNAs.

1 million cells were seeded in 75cm2 treated flask (NUNC™, Roskilde, Denmark) using their corresponding cell culture media. After being incubated for 72hrs, each well was washed with PBS and cells were then trypsinized with 0.05% trypsin in 1X PBS for 15 min, neutralized with soybean trypsin inhibitor (0.1% trypsin inhibitor in 1X PBS) and resuspended in PBS pH 7.4. Cell suspensions were then permeabilized with 10 % MACS Cell Perm Solution (Miltenyi Biotec, Germany) for 5 min and fixed with 10 % MACS Cell Fix Solution (Miltenyi Biotec, Germany) for 30 min followed by washing three times with 1X PBS for 5 min each. Cell pellets were resupended in 1X PBS and spun down onto polylysine-coated glass slides (Sigma-Aldrich, UK) by a cytocentrifuge (Hettich, Germany) at 1,500 rpm for 10 min. Slides were air dried overnight at room temperature and stored at 4°C. Dried slides containing cells immobilized by cytospin were rehydrated with 1X TBS buffer for 5 min and treated twice for 5 min with 100 □l of a 130 mM aqueous solution of 1-methylimidazole (AppliChem, Germany) before surrounding areas of interest with a hydrophobic pen (Dako, Denmark). Then, 100 □l of a 160 mM aqueous solution of 1-ethyl-3-(3-dimethylaminopropyl) carbodiimide (EDC) (Sigma-Aldrich, UK) was added and slides were placed into a humidity chamber (Thermobrite system, Abbot molecular, USA) for 1 hr. Following that time, slides were washed twice for 5 min with 1X PBS before incubating them in a hybridization chamber for 30min with 100 □l of Proteinase QS solution (1:8000 dilution in 1X PBS) (Affymetrix, USA). Slides were then washed twice for 5 min with 1X PBS before being dehydrated with growing ethanol concentrations (70 %, 96 % and 99.5 %) (Sigma Aldrich) for 1 min at each concentration.

### LNA based detection probes.

Locked-Nucleic Acid (LNA) based oligonucleotides to detect miRNA-21, miRNA-200a and U6 snRNA were purchased from Exiqon (Denmark) as miRCURY LNA™ miRNA kits. They are 20-mer oligonucleotides labeled at both 5' and 3' ends with digoxin (DIG).

### Simultaneous detection of cytokeratines and miRNAs by fluorescenct immunocytochemistry and ISH techniques.(MishCTC)

miRNA-21, miRNA-200a and U6 snRNA expression was determined with miRCURY LNA™ miRNA kits (see above). Each probe was independently analyzed. After dehydration process, slides were air dried and incubated with 40 □l of a diluted solution (1:600) of the corresponding LNA miRNA probe, which were pre-denaturized by heating up at 90°C for 4 min, in ISH buffer 1x (Exiqon, Denmark) and hybridized at 58°C in a humidified chamber for 1 h after sealed the samples with fixogum. Following hybridization period and removal of fixogum, slides were washed at 56°C with 5X, 1X and 0.2X SSC for 5min each. Samples were then incubated for 15 min with a blocking solution (0.1% Tween, 2% Sheep serum and 1% BSA in PBS 1x) followed by incubation for 15 min with both FITC-Anti-Cytokeratin antibody (clone: CK3-6H5. Miltenyi Biotec, Germany) and Anti-DIG alkaline fosfatase antibody (Roche Diagnostics, Germany) simultaneously. After incubation with both antibodies, slides were washed with 1X PBS for 5 min. Enzyme-labeled fluorescence (ELF) signal amplification was conducted by applying SIGMAFAST™ Fast Red TR/Naphthol (Sigma-Aldrich, UK) as substrate of alkaline phosphatase activity, diluted in TRIS-hydrochloric buffer according to recommendations of commercial supplier. Finally, Vectashield mounting media with DAPI (Vector Labs. USA) were used to mount the slides.

### Spiking experiments

Spiking experiments were performed in triplicate using 100 MDA-MB 468 cells in 10 ml of twenty venous healthy volunteer blood samples which were collected in 10 ml EDTA tubes (BD, USA). Samples were processed by density gradient centrifugation during 45 min at 400rpm and assisted by Histopaque®-1119 (Sigma-Aldrich, UK) in order to isolate hematopoietic cell fractions, which also contain epithelial cells. Hematopoietic fractions were then incubated for 30 mins with magnetic microbeads labeled with a multi-cytokeratin-specific antibody (CK3-11 D5) (Miltenyi Biotec, Germany) which recognize cytoplasmatic cytokeratin 7,8,18 and 19. Magnetically enriched cell fractions were then passed through MACS Cell Separation magnetic columns (Miltenyi Biotec, Germany) supported at MiniMACS separator (Miltenyi Biotec, Germany) and washed three times with dilution buffer (Miltenyi Biotec, Germany). Magnetic columns were then de-attached from the MiniMACS separator support and cytokeratin positive cells were then eluted from the column after adding dilution buffer and applying pressure. Cytokeratine-positive enriched cell fractions were spun down onto polylysine-coated glass slides. From that point protocols described previously were followed for simultaneous detection of cytokeratines and miRNAs. Recovery rates of tumor cells spiked into normal blood at the low level control numbers were in the range of 60- 75%

### Analysis of CTC from patient samples

25 oncologic metastatic patients were enrolled from the Breast Cancer Unit of the University Hospital of Granada from December 2013 to January 2014. The inclusion criteria were the histological diagnosis of : lung cancer (LC), prostate cancer (PC), colon cancer (CC), urothelial cancer (UC), breast cancer (BC), gastric cancer (GC), ovary cancer (OC) and melanoma cancer (MC) and the availability of tissue for biomarker studies (SI Table 2). The local ethics committee approved this study and eligible patients were selected after the acquisition of informed written consent.

As negative controls, 5 blood samples from healthy volunteers without evidence of an epithelial malignancy were examined. Peripheral blood was drawn from the middle of vein puncture discarding the first 3ml of blood. This precaution was undertaken in order to avoid contamination of the sample with epithelial cells from skin during sample collection to avoid collection of non-tumor epithelial cells. Blood samples were collected in EDTA tubes and transported immediately to laboratory. Then, protocols described in the Spiking Section were performed.

### RNA extraction and reverse transcription and qRT-PCR

Breast cancer cell lines were used to analyze their expression of miRNA-21 and miRNA-200a. Cells were plates (1x103 cells) in 75 cm2 treated flask (NUNC™, Roskilde, Denmark) grew in their culture media for 72 h, the cells were then treated for 3 min with trypsine solution (Sigma Aldrich. US) and then were add 5ml of medium to inhibit the tryptic activity which may damage cells and then the cells were centrifuged at 200 g for 5 min. After the cells were collected in 15 ml tubes washed one once with 1X PBS and then 0.5 ml of miRNA Extraction Kit lysis Mixture (OmegaBio-tek, USA) were added per tube and incubated 5 min at room temperature. Following, 250 µl XD binding buffer were added and incubated on ice for 10 min, after, the solution was add into HiBind® X-press Column and centrifuged for 1 min at 13,000 g. To continue, 1.2 volumes of ethanol were added to the filtrate, and then they were transferred to the HiBind® Micro RNA column and centrifuged again, at 13,000 g for 1 min, the filtrate was discarded and the miRNAs were then recuperated from columns by addition of 500 µl RNA wash buffer. miRNA recuperated from the columns and 5 µl of miRNA extracted were then incubated with 1 µl poly(A) 2 µl tailing buffer and 2 µl of the nuclease free water (Quanta Biosciences USA) and then the molecular solution was incubated for 60 min at 37°C, followed by a 5 min incubation at 70°C. For preparation of RT-PCR, reaction mix was prepared adding 25 µl PerfeCta SYBR Green Supermix, ROX, 200 nM primers and template to 50 µl final volume. RT-PCR in triplicate wells. The reaction was done in 96 well plates in a Real Time PCR system (Applied Biosystems® 7500 Real-Time PCR Systems. USA) for 2 min and 40 cycles of denaturing at 95°C and 60°c for 1 min for annealing and extending at 70°C for 1 min..

### Confocal microscopy

Confocal images were obtained using a ZeissLSM 710 confocal/multiphoton laser scanning microscope equipped with Argon/2 laser (458 nm, 477 nm, 488 nm, 514nm) and a Titanium Sapphire laser (750 nm). The cells were viewed with a 63X (NA1•2) apochromatic water objective and images of different fields were taken. The microscope was setup to take multichannel images and the excitation and emission filter sets configured individually so that there is no fluorescence bleed-through between the channels. The argon (488nm) laser with appropriated emission filter was used for the visualization of FITC. The argon (543nm) laser with appropriated emission filter was used for the visualization of FastRed. FITC was utilized to visualize CK and FastRed/Naphthol was used to visualize each miRNA analyzed. Zen 2009 light edition software (CarlZeiss MicroImaging GmbH) were used to control the microscope, scanning, laser module, and processed of images.

## Claims

1. An *in vitro* method of detecting circulating tumour cells, both circulating tumour cells of epithelial phenotype and circulating tumour cells having Epithelial-mesenchymal transition markers (EMTs), in a biological sample using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumour cells in said biological sample or wherein if the expression levels in the cells of the biological sample are expressed in an amount greater than 2/3 of the maximum expression achieved in a positive control is indicative of the presence of circulating tumour cells in said biological sample.

2. The method of claim 1, wherein the biological sample is any body fluid such as blood or urine.

3. The method of any of claims 1 or 2, wherein the expression levels of miRNA-21 are determined by in-situ hybridization.

4. The method of any of claims 1 to 3, wherein the negative control is a non-tumour epithelial cell or a hematopoietic cell and wherein as used herein overexpression is meant an at least two fold expression level of miRNA-21 in the cells of the biological sample in comparison to the expression level of miRNA-21 in a non-tumour epithelial cell as determined by in situ hybridization or an at least 10 fold expression level of miRNA-21 in the cells of the biological sample in comparison to the expression level of miRNA-21 in a hematopoietic cell, preferably lymphocytes or mononuclear cells, as determined by in situ hybridization.

5. The method of any of claims 1 to 3, wherein the positive control is the epithelial tumor breast cell line (MDA-MB468).

6. The method of any of claims 1 to 6, wherein the biological sample is first treated to isolate the cytokeratin positive cells and/or the EpCAM positive cells and wherein the isolated cytokeratin positive cells and/or the EpCAM positive cells once isolated are use to carry-out the method as defined in any of claims 1-7.

7. The method of claim 6, wherein the cytokeratin positive cells are isolated by immuno-magnetic selection and/or immune-cytochemistry.

8. A method of predicting or prognosticating the progression of cancer in a biological sample of a subject, wherein the subject is suffering from a cancer disease, and wherein the method comprises using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of a malignant progression of said cancer disease or wherein if the expression levels in the cells of the biological sample are expressed in an amount greater than 2/3 of the maximum expression achieved in a positive control is indicative of a malignant progression of said cancer disease.

9. A method of diagnosing cancer in a subject, wherein the method comprises using, as an indicator, expression levels of miRNA-21, and obtaining a result of the method by comparing the expression levels of said miRNA-21 with a negative control or with a positive control, wherein if the expression levels in the cells of the biological sample are over-expressed in comparison to a negative control is indicative of the presence of circulating tumour cells in said biological sample or wherein if the expression levels in the cells of the biological sample are expressed in an amount greater than 2/3 of the maximum expression achieved in a positive control is indicative of the presence of circulating tumour cells in said biological sample.

10. The method of any of claims 8 or 9, wherein the subject is a human subject.

11. The method of claims 8 to 10, wherein the biological sample is any body fluid such as blood or urine.

12. The method of any of claims 8 to 11, wherein the expression levels of miRNA-21 are determined by in-situ hybridization.

13. The method of any of claims 8 to 12, wherein the negative control is a non-tumour epithelial cell or a hematopoietic cell and wherein as used herein overexpression is meant an at least two fold expression level of miRNA-21 in the cells of the biological sample in comparison to the expression level of miRNA-21 in a non-tumour epithelial cell as determined by in situ hybridization or an at least 10 fold expression level of miRNA-21 in the cells of the biological sample in comparison to the expression level of miRNA-21 in a hematopoietic cell, preferably lymphocytes or mononuclear cells, as determined by in situ hybridization.

14. The method of any of claims 8 to 12, wherein the positive control is the epithelial tumor breast cell line (MDA-MB468).

15. The method of any of claims 8 to 14, wherein the biological sample is first treated to isolate the cytokeratin positive cells and/or the EpCAM positive cells and wherein the isolated cytokeratin positive cells and/or the EpCAM positive cells once isolated are use to carry-out the method as defined in any of claims 1-7.

16. The method of claim 15, wherein the cytokeratin positive cells are isolated by immunomagnetic selection and/or immunocytochemistry.

17. The method of any one of claims 8 to 16, wherein the cancer disease is a solid tumour of epithelial origin.

18. The method of the precedent claim, wherein the solid tumour of epithelial origin is selected from the list consisting of ovarian, head and neck, larynx, colon, stomach, prostate, cervix, gastric, urothelial, adrenal, thyroid gland, lung, uterus, rectum, breast or kidney cancer or carcinoma or a sarcoma, melanoma.

19. A method for allocating a human subject suffering from cancer in one of two groups, wherein group 1 comprises subjects identifiable by the method according to claims 8 to 18; and wherein group 2 represents the remaining subjects.

20. A pharmaceutical composition comprising cisplatin and/or other compound acting through the activation of the oxidative stress pathway, for treating a human subject of group 1 as identifiable by the method of claim 19.

21. A pharmaceutical composition comprising platinum coordination complexes, doxorubicin, antracycins, campothecin, bortezomib, procarbazine, cyclophosphamide, adriamycin or alkylating agents, photodynamic therapy and rituximab, for treating a human subject of group 1 as identifiable by the method of claim 19.

22. A kit or a device suitable for carrying out the method of any of claims 1-18, comprising at least one oligonucleotide(s) capable of hybridizing with miRNA-21 and optionally means for the detection of cytokeratin positive cells by immunomagnetic selection and/or immunocytochemistry.

23. The kit or the device of claim 22 which further comprises a positive control sample, optionally a non-tumour epithelial cell.
